# EUROPEAN PATENT APPLICATION

(11) **EP 3 896 700 A1**
(43) Date of publication of application: **20.10.2021**
(21) Application number: 20170160.4
(22) Date of filing: 17.04.2020
(51) Int. Cl.: G16H 20/60, G16H 50/20, G16H 50/70

(54) **IMPROVED METHOD FOR DETERMINING BLOOD GLUCOSE RESPONSES**

(71) Applicant: Perfood GmbH, 23554 Lübeck (DE)
(72) Inventor: TWESTEN, Christoph, 23554 Lübeck (DE); SCHRÖDER, Torsten, 23554 Lübeck (DE); LEITOW, Fynn Marlin, 23554 Lübeck (DE)
(74) Representative: Schrell, Andreas

(57) **Abstract**

The present invention pertains to a method for determining a baseline in a blood glucose curve, a method for determining a blood glucose response of an individual to at least one impact factor, a method for predicting the nutritype of an individual, a method for predicting the blood glucose response of an individual to at least one impact factor, a method for determining personalized lifestyle recommendations for an individual as well as a method for determining the composition of a personalized diet and a method for preparation thereof.

## Description

The present invention pertains to a method for determining a baseline in a blood glucose curve, a method for determining a blood glucose response of an individual to at least one impact factor, a method for predicting the nutritype of an individual, a method for predicting the blood glucose response of an individual to at least one impact factor, a method for determining personalized lifestyle recommendations for an individual as well as a method for determining the composition of a personalized diet and a method for preparation thereof.

The postprandial glycaemic response (PPGR) is a critical factor for metabolic health of humans and for the prevention and treatment of blood glucose related diseases, such as diabetes mellitus type 2 (DMT2). Numerous studies have been conducted to understand the influence of different food products on the PPGR and the health of humans. A high-glycaemic nutrition results in fast increases in blood glucose levels and in response thereto extensive secretion of insulin into the blood stream in order to absorb the glucose into the cells and convert it into glycogen. A problem arising from such excessive peaks in glucose and insulin levels is that absorption of glucose from the blood steam in response to the insulin secretin and associated therewith the reduced blood glucose levels trigger a sensation of hunger although in principle a sufficient amount of energy has already been consumed. The sensation of hunger usually results in repeated food intake, which often leads to an energy intake that exceeds the daily energy requirements of an individual and to a gradual gain of weight. Apart from this, the frequent and excessive secretion of insulin over an extended period of time in response to a high-glycaemic nutrition can lead to a downregulation of insulin receptors on the cell surface and insulin resistance. As a consequence of insulin resistance chronically increased blood glucose levels are observed resulting in various blood glucose related diseases and disorders. Nowadays, a low-glycaemic nutrition and the avoidance of excessive peaks in blood glucose levels is considered to reduce the risk for developing certain chronic diseases and to be beneficial in the treatment of a large number of diseases and disorders such as DMT2, polycystic ovary syndrome (PCOS), migraine, non-alcoholic fatty liver disease (NAFLD) and many more.

Recent studies revealed that the blood glucose level in response to ingestion of a certain food product highly depends on the individual itself, wherein general statements on responses to specific food products cannot be made (Ridaura et al., Gut microbiota from twins discordant for obesity modulate metabolism in mice, 2013, Science, Vol. 341; Zeevi et al., Personalized Nutrition by a Prediction of Glycemic Responses, 2015, Cell, Vol. 163(5)). The blood glucose response to different impact factors, such as to a certain food product, can considerably vary between different individuals. Even within a single individual, the blood glucose response depends on various aspects, such as, but not limited to daytime, general health condition and medication. The determination of the blood glucose response of an individual presently requires precise and individualized measurement of the blood glucose curve in response to a specific impact factor, such as to the consumption of a particular food product or to physical activity, under consideration of possible further interfering impact factors. The analysis and surveillance of the blood glucose response can be accomplished by using continuous blood glucose sensors and an app comprising a diary for various aspects of lifestyle, such as for recording physical activity, food intake, wellness, symptoms, pain, medication, ovulation or sleep. Based on such information, it is possible to determine personalized lifestyle recommendations, in particular a recommendation for a personalized low-glycaemic diet, to maintain or improve the health of an individual or to treat and/or prevent blood glucose related diseases and/or disorders.

At present, the standard for calculating the actual blood glucose response is based on area under the curve (AUC) calculation, which is also used for the determination of the widely used glycaemic index (GI) (Jenkins et al., Metabolic effects of low-glycemic-index diet, 1987, American Journal of Clinical Nutrition, Vol. 46(6)). Comparability of the PPGR to different food products requires a standardization of the calculation method. In various studies different methods and variants for determining the AUC have been compared (Potteiger et al., A comparison of methods for analyzing glucose and insulin areas under the curve following nine months of exercise in overweight adults, 2002, Int J. Obes. Relat. Metab. Disord., Vol. 26(1); Schnell et al., Role of Continuous Glucose Monitoring in Clinical Trials: Recommendations on Reporting, 2017, Diabetes Technol. Ther., Vol. 19(7)). According to these studies, the incremental AUC (iAUC) is suggested as a gold standard for the analysis of blood glucose curves. The blood glucose baseline describes the initial blood glucose level which is used as basis for the calculation of the AUC. It is easy to imagine that a precise and reliable determination of the AUC highly depends on the method of setting the baseline. This is often a difficult and underestimated task, in particular as in the course of the day often strong fluctuations in the blood glucose level of an individual in response to impact factors such as food intake, physical and mental activity, medication, stress, progression of a disease or the sleep pattern can be observed and frequently different factors influencing the blood glucose level are temporally overlapping.

For determination of the baseline of a blood glucose curve, usually either the glucose levels in the last hours of the night, the fasting glucose in the morning, the first determined glucose value or the daily median of the glucose level is used (Zeevi et al., Personalized Nutrition by a Prediction of Glycemic Responses, 2015, Cell, Vol. 163(5)); Brouns et al., 2005, Glycaemic index methodology, Nutrition Research Reviews, Vol. 18, pages 145-171). The use of the glucose levels in the last hours of the night is associated with various disadvantages. Blood glucose levels are influenced by the amount and quality of sleep and measurement accuracy is often negatively affected in dependency on the sleeping position. The fasting glucose in the morning also varies with quality and amount of sleep.

The methods for determining and setting of baselines for blood glucose curves in the prior art are either based on a certain blood glucose level of an individual at a specific time point or on mathematical calculation, such as calculating the average or median of the blood glucose levels measured in a certain time interval. However, these methods do not specifically consider the particular exogenous and endogenous impact factors, in particular the nature, duration and extent of the particular impact factors, causative for the progression of the blood glucose curve of an individual in a given time interval, in particular in cases in which more than one impact factor influences the progression of the blood glucose curve of an individual. Accordingly, the methods for determining a blood glucose baseline as used in the prior art do not provide robust values for correctly setting a blood glucose baseline and, therefore, do not allow precise and reliable determination of the blood glucose response of an individual to ingestion of certain food products and/or to other impact factors.

The present invention overcomes the disadvantages in the prior art by the subject-matter of the independent claims, in particular by providing an improved method for determining a baseline in a blood glucose curve, specifically an improved computer implemented method for determining a baseline in a blood glucose curve.

The present invention pertains to a method for determining a baseline in a blood glucose curve, the method comprising:
a) providing training data comprising blood glucose curves of individuals comprising an impact factor-accounting baseline,
b) subjecting the training data provided in step a) to a machine learning procedure to obtain a trained algorithm for automated determination of baselines in blood glucose curves, and
c) determining a baseline in a blood glucose curve by applying the trained algorithm on a blood glucose curve.

Thus, the present invention in particular pertains to a method of precisely and reliably determining a baseline in a blood glucose curve by using an algorithm that has been trained with training data comprising an impact factor-accounting baseline. Accordingly, the training data provided in step a) comprise blood glucose curves of individuals, wherein each of the blood glucose curves comprises a baseline that has been set taking into account the at least one impact factor affecting the individual and the corresponding progression of the blood glucose curve of the individual in response to the at least one impact factor. Preferably, each of the impact factor-accounting baselines in the blood glucose curves of the training data have specifically been determined under consideration of the at least one factor causative for a deviation of the progression of the blood glucose curve from the blood glucose curve progression which would have been obtained in absence of the at least one impact factor. Accordingly, the impact factor-accounting baselines in the blood glucose curves of the training data are preferably not simply based on the determination of the blood glucose level of an individual at a certain time or constitute the result of an averaging of the blood glucose levels monitored during a predetermined period of time but are in a preferred embodiment individually set based on information on the at least one impact factor causative for the deviation of the progression of the blood glucose curve from the blood glucose curve progression which would have been obtained in absence of the at least one impact factor, in particular on the nature, duration and extent of the at least one impact factor causative for the deviation of the progression of the blood glucose curve from the blood glucose curve progression which would have been obtained in absence of the at least one impact factor. Thus, in a preferred embodiment, the impact factor-accounting baseline is a baseline that has been obtained by assigning the effect of the at least one impact factor to the progression of the blood glucose curve of the individual and by setting a corresponding baseline not being affected by the at least one impact factor. According to the present invention, the impact factor-accounting baselines of the blood glucose curves of the training data have not been solely mathematically determined, in particular have not been determined solely based on blood glucose level measured at a specific time or solely based on averaging of measured blood glucose level. In a particularly preferred embodiment, the impact factor-accounting baselines of the blood glucose curves have been determined based on expert knowledge, in particular have been determined by a qualified expert. In a further preferred embodiment of the present invention, the impact factor-accounting baselines of the blood glucose curves have been determined by a subject or object able to render an independent or new opinion on the accurate progression of the baseline in a blood glucose curve. Preferably, the impact factor-accounting baselines of the blood glucose curves have been determined by a human being or by a computer. According to the present invention, the training data are then subjected to a machine learning procedure in the course of which an algorithm is trained, which can automatically determine a baseline in a blood glucose curve of an individual. The trained algorithm obtained in this way is based on the recognition of certain patterns in a blood glucose curve of an individual. In a next step of the method according to the present invention, the trained algorithm is applied on a blood glucose curve of an individual which does not comprise a blood glucose baseline so as to obtain a blood glucose curve comprising an automatically determined baseline. On the basis of the blood glucose curve of an individual comprising a baseline which has been automatically determined by the trained algorithm, it is advantageously possible to precisely and reliably calculate the blood glucose response of the individual. The determination of the blood glucose response does not suffer from the disadvantages associated with the determination methods for baselines as used in the prior art, in particular by calculating the baseline based on the glucose levels in the last hours of the night, the fasting glucose in the morning, the first determined glucose value or the daily median of the glucose level. In this way, the blood glucose response of an individual can be determined with higher accuracy, in particular also in cases in which more than one impact factor influences the blood glucose level.

In a preferred embodiment of the present invention, the training data provided in step a) comprise long-term blood glucose curves of individuals comprising an impact factor-accounting baseline, in particular blood glucose curves of individuals measured for at least 1 min, preferably at least 5 min, preferably at least 10 min, preferably at least 20 min, preferably at least 30 min, preferably at least 40 min, preferably at least 50 min, preferably at least 1 hour, preferably at least 2 hours, preferably at least 3 hours, preferably at least 4 hours, preferably at least 5 hours, preferably at least 6 hours, preferably at least 7 hours, preferably at least 8 hours, preferably at least 9 hours, preferably at least 10 hours, preferably at least 11 hours, preferably at least 12 hours, preferably at least 13 hours, preferably at least 14 hours, preferably at least 15 hours, preferably at least 16 hours, preferably at least 17 hours, preferably at least 18 hours, preferably at least 19 hours, preferably at least 20 hours, preferably at least 21 hours, preferably at least 22 hours, preferably at least 23 hours, preferably at least 24 hours, comprising an impact factor-accounting baseline.

Particularly preferred, the training data provided in step a) comprise all-day blood glucose curves of individuals comprising an impact factor-accounting baseline.

Preferably, the training data provided in step a) comprise blood glucose curves of individuals in response to at least one impact factor comprising an impact factor-accounting baseline. In a particularly preferred embodiment of the present invention, the training data provided in step a) comprise long-term blood glucose curves of individuals, in particular all-day blood glucose curves of individuals, comprising an impact factor-accounting baseline and blood glucose curves of individuals in response to at least one impact factor comprising an impact factor-accounting baseline.

In a further preferred embodiment of the present invention the trained algorithm for automated determination of baselines in blood glucose curves obtained in step b) is evaluated in a step b1) based on validation data. Preferably, the validation data comprise long-term blood glucose curves of individuals, in particular all-day blood glucose curves of individuals, comprising an impact factor-accounting baseline and/or blood glucose curves of individuals in response to at least one impact factor comprising an impact factor-accounting baseline.

In a preferred embodiment of the present invention, the training data provided in step a) contain at least 10, preferably at least 50, preferably at least 100, preferably at least 250, preferably at least 500, preferably at least 750, preferably at least 1000, blood glucose curves of individuals.

In a preferred embodiment of the present invention, the validation data used in step b1) contain at least 10, preferably at least 50, preferably at least 100, preferably at least 250, preferably at least 500, preferably at least 750, preferably at least 1000, blood glucose curves of individuals.

In a further preferred embodiment, the machine learning procedure is a supervised machine learning procedure.

In a particularly preferred embodiment, the machine learning procedure is based on an algorithm selected from the group consisting of linear regression, logistic regression, support vector machine, decision tree, random forest, K-nearest neighbors (kNN), K-means clustering, naive Bayes, principal component analysis (PCA), supersparse linear integer model (SLIM), neural network.

In a particularly preferred embodiment of the present invention, the determination of a baseline in a blood glucose in step c) is carried out by executing the trained algorithm obtained in step b) on a blood glucose curve of an individual which does not comprise a baseline.

According to further preferred embodiment of the present invention, in step c), the trained algorithm further determines the quality of the automatically determined baseline, in particular determines a coefficient of determination (R²).

Preferably, the coefficient of determination (R²) of the baseline in the blood glucose curve determined in step c) is at least 0.8, preferably at least 0.81, preferably at least 0.82, preferably at least 0.83, preferably at least 0.84, preferably at least 0.85, preferably at least 0.86, preferably at least 0.87, preferably at least 0.88, preferably at least 0.89, preferably at least 0.9, preferably at least 0.91, preferably at least 0.92, preferably 0.93, preferably at least 0.94, preferably at least 0.95, preferably at least 0.96, preferably at least 0.97, preferably at least 0.98, preferably at least 0.99.

In a further preferred embodiment, the coefficient of determination (R²) of the baseline in the blood glucose curve determined in step c) is 0.8 to 1, preferably 0.85 to 1, preferably 0.9 to 1, preferably 0.95 to 1.

The invention further pertains to a method for determining a blood glucose response of an individual to at least one impact factor, in particular a computer implemented method for determining a blood glucose response of an individual to at least one impact factor, the method comprising:
aa) providing at least one blood glucose curve of the individual in response to at least one impact factor,
bb) applying the trained algorithm obtained in step b) of the method for determining a baseline in a blood glucose curve according to the present invention on the at least one blood glucose curve provided in step aa) to obtain at least one blood glucose curve of the individual having an automatically determined baseline, and
cc) analysing the at least one blood glucose curve obtained in step bb) to determine the blood glucose response of an individual to the at least one impact factor.

Accordingly, the method for determining a blood glucose response of an individual to at least one impact factor according to the present invention comprises the provision of at least one blood glucose curve of the individual in response to at least one impact factor in step aa). In a subsequent step bb), the trained algorithm obtained in step b) of the method for determining a baseline in a blood glucose curve according to the present invention is applied on the at least one blood glucose curve of the individual in response to at least one impact factor provided in step aa) to obtain at least one blood glucose curve of the individual having an automatically determined baseline. Finally, in step cc) of the method for determining a blood glucose response of an individual to at least one impact factor, the at least one blood glucose curve obtained in step bb) is analysed to determine the blood glucose response of an individual to the at least one impact factor.

In a particularly preferred embodiment of the present invention, the determination of the blood glucose response in step cc) is based on calculation of the area under the curve (AUC), in particular the incremental area under the curve (iAUC), in particular under consideration of the automatically determined blood glucose baseline.

In a preferred embodiment of the present invention, the determination of the blood glucose response in step cc) is based on calculation of the maximum deviation of the blood glucose curve from the automatically determined blood glucose baseline in response to the at least one impact factor, in particular the maximum increase in the blood glucose level relative to the automatically determined blood glucose baseline in response to the at least one impact factor.

In a further preferred embodiment of the present invention, the determination of the blood glucose response in step cc) is based on calculation of the mathematical derivation of the blood glucose curve in response to the at least one impact factor, in particular of the slope of the blood glucose curve in response to the at least one impact factor.

In a preferred embodiment of the present invention, the blood glucose level of the individual is measured, preferably constantly measured, in particular by using a blood glucose sensor.

Preferably, the blood glucose response of the individual to at least one impact factor affecting the individual is linked to data pertaining to the at least one impact factor, preferably to data provided in a diary for various aspects of lifestyle, in particular information on daytime, age of the individual, sex of the individual, weight of the individual, height of the individual, body mass index (BMI) of the individual, body temperature, microbiota composition in the intestinal tract of the individual, metabolome composition, genome of the individual, type of physical activity, duration of physical activity, type of mental activity, duration of mental activity, type of food, composition of food, amount of food, time of food consumption, health status, type of medication and/or dosage of medication.

In a further preferred embodiment of the present invention, the blood glucose level of the individual is measured, preferably constantly measured, for a predetermined period of time, preferably by using a blood glucose sensor.

Preferably, each blood glucose response of the individual to the at least one impact factor affecting the individual during the predetermined period is linked to data pertaining to the at least one impact factor, preferably to data provided in a diary for various aspects of lifestyle, in particular information on daytime, age of the individual, sex of the individual, weight of the individual, height of the individual, body mass index (BMI) of the individual, body temperature, microbiota composition in the intestinal tract of the individual, metabolome composition, genome of the individual, type of physical activity, duration of physical activity, type of mental activity, duration of mental activity, type of food, composition of food, amount of food, time of food consumption, health status, type of medication and/or dosage of medication.

In a preferred embodiment, the blood glucose level of the individual is measured in intervals of 30 seconds, preferably 1 minute, preferably 2 minutes, preferably 3 minutes, preferably 4 minutes, preferably 5 minutes, preferably 6 minutes, preferably 7 minutes, preferably 8 minutes, preferably 9 minutes, preferably 10 minutes, preferably 15 minutes.

Preferably, the blood glucose level of the individual is measured at least every 15 minutes, preferably at least every 10 minutes, preferably at least every 9 minutes, preferably at least every 8 minutes, preferably at least every 7 minutes, preferably at least every 6 minutes, preferably every 5 minutes, preferably every 4 minutes, preferably every 3 minutes, preferably every 2 minutes, preferably every minute, preferably every 30 seconds.

In a further preferred embodiment of the present invention, the predetermined period of time is at least 1 day, preferably at least 2 days, preferably at least 3 days, preferably at least 4 days, preferably at least 5 days, preferably at least 6 days, preferably at least 7 days, preferably at least 8 days, preferably at least 9 days, preferably at least 10 days, preferably at least 11 days, preferably at least 12 days, preferably at least 13 days, preferably at least 14 days, preferably at least 3 weeks, preferably at least 4 weeks, preferably at least 1 month, preferably at least 2 months, preferably at least 3 months, preferably at least 4 months, preferably at least 6 months, preferably at least 8 months, preferably, at least 10 months, preferably at least 12 months.

In a further preferred embodiment of the present invention, the blood glucose response of the individual to the at least one impact factor determined in step cc) is included into a database comprising blood glucose responses of the individual to different impact factors, preferably into a database comprising blood glucose responses of different individuals to different impact factors, in particular a database comprising blood glucose responses of different individuals classified into the same nutritype to different impact factors.

The present invention further relates to a method for predicting the nutritype of an individual, in particular a computer implemented method for predicting the nutritype of an individual, the method comprising:
i) providing at least one blood glucose response of an individual to at least one impact factor obtained by the method for determining a blood glucose response of an individual to at least one impact factor of the present invention,
ii) assigning the blood glucose response of the individual to the at least one impact factor to a group of blood glucose responses of different individuals to the at least one impact factor in a database using a nutritype classification model, and
iii) outputting a nutritype of the individual based on the at least one blood glucose response of the individual to the at least one impact factor.

According to the present invention, the groups of blood glucose responses of different individuals to the at least one impact factor in a database correspond to different nutritypes.

In a preferred embodiment of the present invention, the blood glucose responses to the at least one impact factor, in particular the AUC, preferably the iAUC, the maximum increase of the blood glucose level relative to the automatically determined blood glucose baseline and/or the slope of the blood glucose curve in response to the at least one impact factor, within the groups of blood glucose responses of different individuals to the at least one impact factor in the database of step ii) vary from each other by at most 40%, preferably at most 35%, preferably at most 30%, preferably at most 25%, preferably at most 20%, preferably at most 18%, preferably at most 16%, preferably at most 15%, preferably at most 14%, preferably at most 13%, preferably at most 12%, preferably at most 11%, preferably at most 10%, preferably at most 9%, preferably at most 8%, preferably at most 7%, preferably at most 6%, preferably at most 5%, preferably at most 4%, preferably at most 3%, preferably at most 2%, preferably at most 2%, preferably at most 1%.

In a further preferred embodiment of the present invention, the at least one blood glucose response of the individual to at least one impact factor in step i) is linked to data on food intake, in particular data on the type, composition and amount of the food consumed. Preferably, the at least one blood glucose response of the individual to at least one impact factor in step i) is linked to data on at least one further impact factor, in particular to data on at least one individual-specific impact factor.

The present invention also pertains to a method for predicting the blood glucose response of an individual to at least one impact factor, in particular a computer implemented method for predicting the blood glucose response of an individual to at least one impact factor, the method comprising:
x) providing data pertaining to at least one impact factor,
y) assigning the data pertaining to at least one impact factor to at least one blood glucose response in a database comprising blood glucose responses of the individual to different impact factors, preferably comprising blood glucose responses of different individuals to different impact factors, in particular comprising blood glucose responses of different individuals classified into the same nutritype to different impact factors, obtained by the method for determining a blood glucose response of an individual to at least one impact factor according to the present invention, and
z) outputting a predicted blood glucose response of the individual to the at least one impact factor.

In step x) of the method for predicting the blood glucose response of an individual to at least one impact factor according to the present invention, data on the at least one impact factor in question is provided. Preferably, the data comprises information on daytime, age of the individual, sex of the individual, weight of the individual, height of the individual, body mass index (BMI) of the individual, body temperature, microbiota composition in the intestinal tract of the individual, metabolome composition, genome of the individual, type of physical activity, duration of physical activity, type of mental activity, duration of mental activity, type of food, composition of food, amount of food, time of food consumption, health status, type of medication and/or dosage of medication. In subsequent step y), this data is assigned to at least one blood glucose response in a database comprising blood glucose responses of the individual to different impact factors, preferably comprising blood glucose responses of different individuals to different impact factors, in particular comprising blood glucose responses of different individuals classified into the same nutritype to different impact factors, wherein the blood glucose responses in the database have been obtained by the method for determining a blood glucose response of an individual to at least one impact factor according to the present invention and therefore have been determined on the basis of a blood glucose curve of the individual having an automatically determined baseline. On the basis of the assignment of the data pertaining to at least one impact factor to a specific blood glucose response of the individual to the at least one impact factor, preferably to a specific blood glucose response of a different individual to the at least one impact factor, in particular to a specific blood glucose response of a different individual classified into the same nutritype to the at least one impact factor, a prediction of the blood glucose response of the individual to the at least one impact factor is made and indicated to the individual. The predicted blood glucose response advantageously enables the individual to estimate the influence, in particular the extent and duration, of a single impact factor or a combination of various impact factors on the individual's blood glucose level. In this way, an individual can e.g. estimate the influence of consumption of a specific food at a given time on the extent and duration of the blood glucose level increase.

In case the database does not comprise a blood glucose response of the individual to the at least one impact factor in question, preferably a blood glucose response of the individual to the at least one impact factor in question or a blood glucose response of a different individual to the at least one impact factor in question, in particular a blood glucose response of a different individual classified into the same nutritype to the at least one impact factor in question, an assignment of the data to a blood glucose response of the individual to at least one impact factor, preferably to a blood glucose response of a different individual to at least one impact factor, in particular a blood glucose response of a different individual classified into the same nutritype to at least one impact factor, is made, wherein the at least one impact factor is comparable to the at least one impact factor in question based on the data pertaining to the at least one impact factor provided in step x). In a particularly preferred embodiment, comparable impact factors are such which the skilled person would consider to have the most in common with the at least one impact factor in question. According to this particular embodiment, it is conceivable that the data pertaining to the impact factor in question contain the information that 200 grams of potatoes have been ingested at 5 p.m. The database, however, comprises e.g. a blood glucose response of the individual, preferably a blood glucose response of a different individual, in particular a blood glucose response of a different individual classified into the same nutritype, to the ingestion of 250 grams of potatoes at 6 p.m. In case the database does not contain any blood glucose response of the individual, preferably any blood glucose response of a different individual, in particular any blood glucose response of a different individual classified into the same nutritype, to the at least one impact factor in question, of which the corresponding at least one impact factor is closer to the at least one impact factor in question, in particular has more in common with the at least one impact factor in question, then said particular blood glucose response of the individual, preferably of a different individual, in particular a different individual classified into the same nutritype, in the database is used for the prediction of the blood glucose response of the individual to the at least one impact factor in question, in particular for the prediction of the blood glucose response of the individual to ingestion of 200 gram of potatoes at 5 p.m.

In a preferred embodiment of the present invention, the blood glucose responses of different individuals to different impact factors in the database are assigned to specific nutritypes of the individuals. According to this particular embodiment, the database comprises groups of blood glucose responses of different individuals assigned to the same nutritype.

In a preferred embodiment of the present invention, the database of step y) comprises blood glucose responses of different individuals to different impact factors, in particular blood glucose responses of different individuals classified into the same nutritype to different impact factors, obtained by the method for determining a blood glucose response of an individual to at least one impact factor according to the present invention.

Accordingly, the database of step y) can comprise blood glucose responses of the individual to different impact factors and/or blood glucose responses of different individuals to different impact factors, in particular blood glucose responses of different individuals classified into the same nutritype to different impact factors.

In a further preferred embodiment of the present invention, the database of step y) consists of blood glucose responses of the individual to different impact factors and/or blood glucose responses of different individuals to different impact factors, in particular blood glucose responses of different individuals classified into the same nutritype to different impact factors.

In a particularly preferred embodiment of the present invention, the blood glucose responses of the individual to different impact factors and/or blood glucose responses of different individuals to different impact factors, in particular blood glucose responses of different individuals classified into the same nutritype to different impact factors in the database of step y) are each linked to data on the at least one impact factor, in particular to data comprising information on daytime, age of the individual, sex of the individual, weight of the individual, height of the individual, body mass index (BMI) of the individual, body temperature, microbiota composition in the intestinal tract of the individual, metabolome composition, genome of the individual, type of physical activity, duration of physical activity, type of mental activity, duration of mental activity, type of food, composition of food, amount of food, time of food consumption, health status, type of medication and/or dosage of medication.

In a preferred embodiment of the present invention, the blood glucose responses of different individuals to different impact factors in the database of step y) are assigned to at least two nutritypes, preferably at least three nutritypes, preferably at least four nutritypes, preferably at least five nutritypes, preferably at least six nutritypes, preferably at least seven nutritypes, preferably at least eight nutritypes, preferably at least nine nutritypes, preferably at least 10 nutritypes, preferably at least 15 nutritypes, preferably at least 20 nutritypes.

In a particularly preferred embodiment of the present invention, in a step x1) preceding step y) the individual is classified into a specific nutritype based on a nutritype classification model, in particular by the method for predicting the nutritype of an individual according to the present invention.

Preferably, in step x1) the individual is classified into a specific nutritype based on a nutritype classification model, in particular by the method for predicting the nutritype of an individual according to the present invention, assigning at least one blood glucose response of the individual to at least one impact factor to a group of comparable blood glucose responses of different individuals to the at least one impact factor in the database of step y).

In a further preferred embodiment, in step x1) the individual is classified into a specific nutritype based on a nutritype classification model, in particular by the method for predicting the nutritype of an individual according to the present invention, assigning data on at least one individual-specific impact factor to individuals having at least one identical individual-specific impact factor, preferably at least two identical individual-specific impact factors, preferably at least three identical individual-specific impact factor, preferably at least three identical individual-specific impact factors, preferably at least four identical individual-specific impact factors, preferably at least five identical individual-specific impact factors.

In a particularly preferred embodiment of the present invention, in step x1) the individual is classified into a specific nutritype based on a nutritype classification model, in particular by the method for predicting the nutritype of an individual according to the present invention, assigning data on at least one individual-specific impact factor to individuals having at least one comparable individual-specific impact factor, preferably at least two comparable individual-specific impact factors, preferably at least three comparable individual-specific impact factor, preferably at least three comparable individual-specific impact factors, preferably at least four comparable individual-specific impact factors, preferably at least five comparable individual-specific impact factors.

In a further preferred embodiment of the present invention, the nutritype classification model is obtained by a machine learning procedure, preferably by a supervised machine learning procedure, preferably by an unsupervised machine learning procedure.

Preferably, the machine learning procedure is based on an algorithm selected from the group consisting of linear regression, logistic regression, support vector machine, decision tree, random forest, K-nearest neighbors (kNN), K-means clustering, naive Bayes, principal component analysis (PCA), supersparse linear integer model (SLIM), neural network.

Preferably, the comparable blood glucose responses of different individuals to the at least one impact factor, in particular the comparable blood glucose responses of different individuals classified into a specific nutritype to the at least one impact factor, have at least 50% identity, preferably at least 55% identity, preferably at least 60% identity, preferably at least 65% identity, preferably at least 70% identity, preferably at least 75% identity, preferably at least 80% identity, preferably at least 85% identity, preferably at least 90% identity, preferably at least 91% identity, preferably at least 92% identity, preferably at least 93% identity, preferably at least 94% identity, preferably at least 95% identity, preferably at least 96% identity, preferably at least 97% identity, preferably at least 98% identity, preferably at least 99% identity, preferably at least 99,5% identity, with the blood glucose responses of the individual to the at least one impact factor.

In a particularly preferred embodiment of the present invention, in step y), the data pertaining to at least one impact factor provided in step x) are assigned to at least one blood glucose response in a database comprising blood glucose responses of different individuals to different impact factors, in particular in a database comprising blood glucose responses of different individuals classified into the same nutritype to different impact factors, obtained by the method for determining a blood glucose response of an individual to at least one impact factor according to the present invention.

In a further preferred embodiment, the database in step y) is extended, preferably successively extended, with blood glucose response of the individual to specific impact factors determined by the method for determining a blood glucose response of an individual to at least one impact factor according to the present invention.

Preferably, the database in step y) is extended, preferably successively extended, with blood glucose response of different individuals to specific impact factors, in particular with blood glucose responses of different individuals classified into the same nutritype to specific impact factors, determined by the method for determining a blood glucose response of an individual to at least one impact factor according to the present invention.

In a preferred embodiment of the present invention, the at least one impact factor is selected from food intake, physical activity, mental activity, medication, sleep or a combination thereof.

In a further preferred embodiment of the present invention, the data pertaining to at least one impact factor provided in step x) include information on daytime, age of the individual, sex of the individual, weight of the individual, height of the individual, body mass index (BMI) of the individual, body temperature, microbiota composition in the intestinal tract of the individual, metabolome composition, genome of the individual, type of physical activity, duration of physical activity, type of mental activity, duration of mental activity, type of food, composition of food, amount of food, time of food consumption, health status, type of medication and/or dosage of medication.

In a preferred embodiment of the present invention, the data pertaining to food intake include information on daytime, age of the individual, sex of the individual, weight of the individual, height of the individual, body mass index (BMI) of the individual, body temperature, microbiota composition in the intestinal tract of the individual, metabolome composition, genome of the individual, type of physical activity, duration of physical activity, type of mental activity, duration of mental activity, type of food, composition of food, amount of food, time of food consumption, health status, type of medication and/or dosage of medication.

In a further preferred embodiment of the present invention, the data pertaining to physical activity include information on daytime, age of the individual, sex of the individual, weight of the individual, height of the individual, body mass index (BMI) of the individual, body temperature, microbiota composition in the intestinal tract of the individual, metabolome composition, genome of the individual, type of physical activity, duration of physical activity, type of mental activity, duration of mental activity, health status, type of medication and/or dosage of medication.

In a preferred embodiment of the present invention, the data pertaining to mental activity include information on daytime, age of the individual, sex of the individual, weight of the individual, height of the individual, body mass index (BMI) of the individual, body temperature, microbiota composition in the intestinal tract of the individual, metabolome composition, genome of the individual, type of physical activity, duration of physical activity, type of mental activity, duration of mental activity, health status, type of medication and/or dosage of medication.

In a further preferred embodiment of the present invention, the data pertaining to medication include information on daytime, age of the individual, sex of the individual, weight of the individual, height of the individual, body mass index (BMI) of the individual, body temperature, microbiota composition in the intestinal tract of the individual, metabolome composition, genome of the individual, type of physical activity, duration of physical activity, type of mental activity, duration of mental activity, health status, type of medication and/or dosage of medication.

In a further preferred embodiment of the present invention, the data pertaining to sleep include information on daytime, age of the individual, sex of the individual, weight of the individual, height of the individual, body mass index (BMI) of the individual, body temperature, microbiota composition in the intestinal tract of the individual, metabolome composition, genome of the individual, health status, type of medication and/or dosage of medication.

In a preferred embodiment of the present invention, the database comprises at least at least 10, preferably at least 50, preferably at least 100, preferably at least 250, preferably at least 500, preferably at least 750, preferably at least 1000, preferably at least 2000, preferably at least 3000, blood glucose responses of the individual to different impact factors.

In a further preferred embodiment of the present invention, the database comprises at least at least 10, preferably at least 50, preferably at least 100, preferably at least 250, preferably at least 500, preferably at least 750, preferably at least 1000, preferably at least 2000, preferably at least 3000, blood glucose responses of different individuals to different impact factors, in particular blood glucose responses of different individuals classified into the same nutritype to different impact factors.

Preferably, the database comprises blood glucose responses of the individual, preferably blood glucose responses of different individuals, in particular blood glucose responses of different individuals classified into the same nutritype, to at least two different impact factors, preferably to at least three different impact factors, preferably to at least four different impact factors, preferably to at least five different impact factors, preferably to at least six different impact factors, preferably to at least seven different impact factors, preferably to at least eight different impact factors, preferably to at least nine different impact factors, preferably to at least 10 different impact factors, preferably to at least 15 different impact factors, preferably to at least 20 different impact factors, preferably to at least 25 different impact factors, preferably to at least 30 different impact factors, preferably to at least 35 different impact factors, preferably to at least 40 different impact factors, preferably to at least 45 different impact factors, preferably to at least 50 different impact factors, preferably to at least 75 different impact factors, preferably to at least 100 different impact factors, preferably to at least 150 different impact factors, preferably to at least 200 different impact factors, preferably to at least 250 different impact factors, preferably to at least 500 different impact factors, preferably to at least 1000 different impact factors, preferably to at least 2000 different impact factors.

In a further preferred embodiment of the present invention, the database comprising blood glucose responses of the individual to different impact factors, preferably the database comprising blood glucose responses of different individuals to different impact factors, in particular the database comprising blood glucose responses of different individuals classified into the same nutritype to different impact factors, is locally stored, in particular stored on a computer-readable storage medium.

In another preferred embodiment of the present invention, the database comprising blood glucose responses of the individual to different impact factors, preferably the database comprising blood glucose responses of different individuals to different impact factors, in particular the database comprising blood glucose responses of different individuals classified into the same nutritype to different impact factors, is globally stored, in particular stored on a server.

In a preferred embodiment of the present invention, the assignment of the data pertaining to at least one impact factor provided in step x) to at least one blood glucose response in a database comprising blood glucose responses of the individual to different impact factors, preferably to at least one blood glucose response in a database comprising blood glucose responses of different individuals to different impact factors, in particular to at least one blood glucose response in a database comprising blood glucose responses of different individuals classified into the same nutritype to different impact factors, in step y) is performed by a blood glucose response classification model, preferably a blood glucose response classification model obtained by a machine learning procedure.

Preferably, the machine learning procedure is an unsupervised machine learning procedure. In a further embodiment of the present invention the machine learning procedure is a supervised machine learning procedure.

Preferably, the machine learning procedure is based on an algorithm selected from the group consisting of linear regression, logistic regression, support vector machine, decision tree, random forest, K-nearest neighbors (kNN), K-means clustering, naive Bayes, principal component analysis (PCA), supersparse linear integer model (SLIM), neural network.

The invention further pertains to a method for determining personalized lifestyle recommendations for an individual, in particular a computer implemented method for determining personalized lifestyle recommendations for an individual, the method comprising the steps:
i) providing a database comprising blood glucose responses of the individual to different impact factors, preferably a database comprising blood glucose responses of different individuals to different impact factors, in particular a database comprising blood glucose responses of different individuals classified into the same nutritype to different impact factors, determined by the method for determining a blood glucose response of an individual to at least one impact factor according to the present invention,
ii) providing data pertaining to at least one impact factor affecting the individual,
iii) calculating personalized lifestyle recommendations for the individual based on the blood glucose response of the individual to the at least one impact factor affecting the individual in the database provided in step i), preferably based on the blood glucose responses of different individuals to the at least one impact factor affecting the individual in the database provided in step i), in particular based on blood glucose responses of different individuals classified into the same nutritype to the at least one impact factor affecting the individual in the database provided in step i), and
iv) outputting personalized lifestyle recommendations for the individual.

The method for determining personalized lifestyle recommendations advantageously allows to provide an individual with a behavioural recommendation in order to avoid excessive variations or peaks in the blood glucose levels of the individual, in particular to avoid the occurrence of excessive peaks in the blood glucose curve and associated with it excessive variations or peaks of insulin and other hormones or hormonal active biomolecules and small molecules, such as (neuro-)peptides, saccharides, lipids, fatty acids, neurotransmitters, metabolites, or nucleic acids. In a preferred embodiment of the present invention, the personalized lifestyle recommendations cover various aspect of life, such as, but not limited to nutritional behaviour, physical activity and sleeping behaviour. In a particular preferred embodiment of the present invention, the personalized lifestyle recommendations are selected from the group consisting of a personalized diet, a personalized training plan, a personalized medication plan, personalized sleep recommendations and/or personalized spiritual exercises, such as meditation. The personalized lifestyle recommendations determined by the method according to the present invention advantageously consider the fact that the blood glucose response of different individuals to specific impact factors can considerably vary. Accordingly, the personalized lifestyle recommendations obtained in step iv) provide an individual with a behavioural recommendation for avoiding excessive variations or peaks in the blood glucose levels of the individual, preferably for maintaining or improving the health of an individual and/or for treating and/or preventing blood glucose related diseases and/or disorders.

In a preferred embodiment of the present invention, the method for determining personalized lifestyle recommendations is a method for determining the composition of a personalized diet. According to said particular embodiment, the method comprises the steps:
i) providing a database comprising blood glucose responses of the individual to different impact factors, preferably a database comprising blood glucose responses of different individuals to different impact factors, in particular a database comprising blood glucose responses of different individuals classified into the same nutritype to different impact factors, determined by the method for determining a blood glucose response of an individual to at least one impact factor according to the present invention,
ii) providing data pertaining to at least one impact factor affecting the individual,
iii) calculating the composition of a personalized diet based on the blood glucose response of the individual to the at least one impact factor affecting the individual in the database provided in step i), preferably based on the blood glucose responses of different individuals to the at least one impact factor affecting the individual in the database provided in step i), in particular based on blood glucose responses of different individuals classified into the same nutritype to the at least one impact factor affecting the individual in the database provided in step i), and
iv) outputting the composition of a personalized diet.

Most preferably, the method for determining the composition of a personalized diet provides an individual with a dietary recommendation for a low-glycaemic nutrition, in particular with a personalized diet for use in the treatment and/or prevention of blood glucose related diseases and/or disorders.

In a particularly preferred embodiment, a selection of at least two, preferably at least three, preferably at least four, preferably at least five, different compositions of a personalized diet is provided in step iv).

Preferably, the personalized diet comprises at least one meal, preferably at least two meals, preferably at least three meals.

The invention further pertains to a computer program product, directly loadable into the internal memory of a digital computer, comprising software code portions which, when the program is executed by a computer cause the computer to carry out i) the method for determining a baseline in a blood glucose curve in response to at least one impact factor according to the present invention, ii) the method for determining a blood glucose response of an individual to at least one impact factor according to the present invention, iii) the method for predicting the nutritype of an individual according to the present invention, iv) the method for predicting the blood glucose response of an individual to at least one impact factor according to the present invention, and/or v) the method for determining personalized lifestyle recommendations for an individual according to the present invention, in particular the method for determining the composition of a personalized diet according to the present invention.

The invention further relates to a computer-readable storage medium comprising software code portions, which when executed by a computer cause the computer to carry out i) the method for determining a baseline in a blood glucose curve in response to at least one impact factor according to the present invention, ii) the method for determining a blood glucose response of an individual to at least one impact factor according to the present invention, iii) the method for predicting the nutritype of an individual according to the present invention, iv) the method for predicting the blood glucose response of an individual to at least one impact factor according to the present invention, and/or v) the method for determining personalized lifestyle recommendations for an individual according to the present invention, in particular the method for determining the composition of a personalized diet according to the present invention.

The invention further relates to a device comprising:
- a display unit, displaying a user interface,
- an input unit,
- a memory unit, and
- a processing unit,
wherein the memory unit comprises a computer program product according to the present invention, in particular a computer program product comprising software code portions which, when the program is executed by the processing unit cause the derive to carry out i) the method for determining a baseline in a blood glucose curve in response to at least one impact factor according to the present invention, ii) the method for determining a blood glucose response of an individual to at least one impact factor according to the present invention, iii) the method for predicting the nutritype of an individual, iv) the method for predicting the blood glucose response of an individual to at least one impact factor according to the present invention, and/or v) the method for determining personalized lifestyle recommendations for an individual according to the present invention, in particular the method for determining the composition of a personalized diet according to the present invention.

In a preferred embodiment of the present invention, the device is a mobile device, in particular a battery-powered wireless mobile device. Preferably, the mobile device, in particular the battery-powered wireless mobile device, is selected from the group consisting of tablet computers, smartphones, smart watches and fitness tracking devices.

In a further preferred embodiment of the present invention the device, preferably mobile device, in particular battery-powered wireless mobile device, is able to establish a connection, in particular wireless connection, to a server on which a database, in particular a database comprising blood glucose responses of an individual to different impact factors, preferably a database comprising blood glucose responses of different individuals to different impact factors, in particular a database comprising blood glucose responses of different individuals classified into the same nutritype to different impact factors, is stored.

Preferably, the device, preferably mobile device, in particular battery-powered wireless mobile device, is able to introduce blood glucose responses of an individual in response to at least one impact factor, in particular blood glucose responses of an individual determined by the method for determining a blood glucose response of an individual to at least one impact factor according to the present invention, into a database comprising blood glucose responses of the individual to different impact factors, preferably a database comprising blood glucose responses of different individuals to different impact factors, in particular a database comprising blood glucose responses of different individuals classified into the same nutritype to different impact factors, stored on a server.

Thus, in a particularly preferred embodiment of the present invention, the device, preferably mobile device, in particular battery-powered wireless mobile device, is able to access and edit a database comprising blood glucose responses of the individual to different impact factors, preferably a database comprising blood glucose responses of different individuals to different impact factors, in particular a database comprising blood glucose responses of different individuals classified into the same nutritype to different impact factors, globally stored on a server.

In a further preferred embodiment of the present invention, the device, preferably desktop device or mobile device, in particular battery-powered wireless mobile device, is able to access and edit a database comprising blood glucose responses of the individual to different impact factors, preferably a database comprising blood glucose responses of different individuals to different impact factors, in particular a database comprising blood glucose responses of different individuals classified into the same nutritype to different impact factors, locally stored in the memory unit of the device.

The invention also pertains to a method for preparing a personalized diet, the method comprising the steps:
xx) determining the composition of a personalized diet according to the method for determining a composition of a personalized diet according to the present invention, and
yy) preparing the components of the personalized diet having the composition determined in step xx).

The invention further pertains to a personalized diet obtained by the method for preparing a personalized diet for use in the treatment of blood glucose related diseases and/or disorders.

In a preferred embodiment of the present invention, the blood glucose related disease and/or disorder is selected from the group consisting of diabetes mellitus type 1 (DMT1), diabetes mellitus type 2 (DMT2), gestational diabetes, hyperglycaemia, metabolic syndrome, cardiovascular diseases, glucose intolerance, polycystic ovary syndrome (PCOS), migraine, non-alcoholic fatty liver disease (NAFLD), cancer, acne, atopic dermatitis, psoriasis, rosacea, atrial fibrillation, dyslipidaemia, HIV, arterial hypertension, pre-diabetes, obesity, brain/cognitive dysfunction, Alzheimer's disease, depression, symptoms of menopause, menstrual dysregulation, cartilage damage, Parkinson's disease, rheumatic diseases, chronic inflammation.

The invention also pertains to the use of a personalized diet obtained by the method for preparing a personalized diet according to the present invention in induction of pregnancy, regulation of menstrual cycle, weight loss, anti-aging or in treatment and/or prevention of menstrual problems and symptoms of menopause.

In the context of the present invention, the term "blood glucose curve" refers to the concentration of glucose in the blood in a time dependent manner. The term "blood glucose level" describes the concentration of glucose in the blood at a given time. In the context of the present invention, the term "blood glucose response" often also referred to as "glycaemic response" designates the time dependent progression of the blood glucose curve in response to at least one specific impact factor under consideration of the blood glucose baseline of an individual. The "blood glucose response" preferably encompasses information on the exact time dependent progression and extent of the blood glucose curve assignable to at least one particular impact factor. In a particularly preferred embodiment, the "blood glucose response" corresponds to the area under curve (AUC), in particular the incremental area under the curve (iAUC), which is bordered by the blood glucose baseline, the maximum increase in the blood glucose level relative to the automatically determined blood glucose baseline or the slope of the blood glucose curve.

In the context of the present invention, the term "impact factor" relates to any exogenous and endogenous factor suitable to influence the blood glucose level of an individual, in particular suitable to trigger a blood glucose response of an individual. The term "individual-specific impact factor" as used in the context of the present invention relates to specific endogenous factors of an individual suitable to influence the blood glucose level, such as, but not limited to age of the individual, sex of the individual, weight of the individual, height of the individual, body mass index (BMI) of the individual, body temperature, intestinal microbiota composition, metabolome composition, genome of the individual.

The term "algorithm" designates a sequence of well-defined unambiguous computer-implementable instructions to perform a specific task.

In the context of the present invention, the expression "nutritype classification model" designates a classification model classifying an individual into a specific nutritype.

The expression "blood glucose response classification model" as used in the context of the present invention relates to a classification model assigning data pertaining to at least one impact factor to at least one blood glucose response in a database comprising blood glucose responses of the individual to different impact factors, preferably comprising blood glucose responses of different individuals to different impact factors, in particular blood glucose responses of different individuals classified into the same nutritype to different impact factors.

In the context of the present invention, the term "nutritype" designates a defined group of individuals which are characterized by a comparable blood glucose response to specific impact factors, in particular to a group of individuals which are characterized by a comparable metabolism. Preferably, the individuals of a specific "nutritype" are characterized by a similar genotype, are epigenetically similar and/or have comparable compositions of the intestinal microbiome.

In the context of the present invention, the expression "blood glucose curve having an impact factor-accounting baseline" designates a blood glucose curve comprising a baseline, which has been determined under consideration of the at least one impact factor causative for a deviation of the progression of the blood glucose curve from the blood glucose curve progression which would have been obtained in absence of the at least one impact factor. Particularly, an "impact factor-accounting baseline" is in contrast to the blood glucose curve baselines of the prior art preferably not simply based on the determination of the blood glucose level of an individual at a certain time or constitutes the result of averaging of the blood glucose levels monitored during a predetermined time period but is preferably individually set based on information on the at least one impact factor causative for the deviation of the progression of the blood glucose curve from the blood glucose curve progression which would have been obtained in absence of the at least one impact factor, in particular on the nature, duration and extent of the at least one impact factor causative for the deviation of the progression of the blood glucose curve from the blood glucose curve progression which would have been obtained in absence of the at least one impact factor. Preferably, the impact factor-accounting baseline is a baseline that has been obtained by assigning the effect of the at least one impact factor to the progression of the blood glucose curve of the individual and by setting a corresponding baseline not being affected by the at least one impact factor. The "impact factor-accounting baseline" according to the present invention is a baseline, which is not solely mathematically determined, in particular which is not solely determined based on a blood glucose level measured at a specific time or solely based on averaging of measured blood glucose level. Preferably, the "impact factor-accounting baseline" is a baseline, which has been determined based on expert knowledge, in particular has been determined by a qualified expert. In a further preferred embodiment of the present invention, the "impact factor-accounting baselines" of the blood glucose curves have been determined by a subject or object able to render an independent or new opinion on the accurate progression of the baseline in a blood glucose curve. Preferably, the "impact factor-accounting baselines" of the blood glucose curves have been determined by a human being or by a computer. According to the present invention, the conduction of the analysis and/or the determination, in particular of the non-mathematical analysis and/or determination, of a baseline in a blood glucose curve based on expert knowledge, in particular based on human expertise, *as such* is not part of the present invention. The methods according to the present invention are based on the use of training data comprising blood glucose curves of individuals comprising an "impact factor-accounting baseline" which has previously been determined, in particular not solely mathematically determined, wherein these training data are used for training an algorithm for automated baseline determination by employing a machine learning procedure.

In the context of the present invention, the terms "food" and "food product" encompass any raw and prepared comestible product, such as specific fruits, vegetables, meat, fish, as well as combinations of different raw and prepared comestible products, such as bread, breakfast meals, pasta dishes, salads, sauces, beverages, confectionery, candy. The terms further relate to at least one micro- or macro-nutrient and combinations of micro- and macro-nutrients.

In the context of the present invention the term "personalized" pertains to an individual-specific adaptation, in particular an individual-specific adaptation considering the exogenous and endogenous factors affecting a particular individual. Accordingly, the term "personalized diet" as used in the context of the present invention relates to a nutrition specifically adapted to a particular individual under consideration of the exogenous and endogenous factors affecting the individual.

The term "personalized lifestyle recommendations" pertains to tailored recommendations for an individual's way or style of life, in particular for the behaviour of the individual. In the context of the present invention the term may cover various aspect of life, such as, but not limited to nutritional behaviour, physical activity and sleeping behaviour. The "personalized lifestyle recommendations" according to the present invention particularly aims to avoid excessive variations or peaks in the blood glucose levels of the individual.

The term "personalized diet" encompasses a personalized single meal, but also a recommendation of various personalized meals to be preferably consumed during a day, week or month. The term further relates to personalized compositions of micro- and macro-nutrients.

In the context of the present invention the term "computer-readable storage medium" includes any machine readable medium, in particular computer storage media and communication media including any medium that facilitates transfer of a computer program from one place to another. A storage media may be any available media that can be accessed and read by a computer. By way of example, and not limitation, such computer-readable media can comprise random access memory (RAM), read-only memory (ROM), electrically erasable programmable read-only memory (EEPROM), or other optical disk storage, semiconductor memory, magnetic disk storage or any other medium that can be used to carry or store desired program code in the form of instructions or data structures and that can be accessed by a computer. Also, any connection is properly termed a computer-readable medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, or wireless technologies such as infrared, radio, and microwave are included in the definition of medium. Disk and disc, as used herein, includes compact disc (CD), laser disc, optical disc, digital versatile disc (DVD), floppy disk and blu-ray disc, wherein disks usually reproduce data magnetically, while discs reproduce data optically with lasers.

In the context of the present invention, the term "a" is meant to include the meaning of "one" or "one or more".

In the context of the present invention, the term "comprising" preferably has the meaning of "containing" or "including", meaning including the specifically identified elements without excluding the presence of further elements. However, in a preferred embodiment, the term "comprising" is also understood to have the meaning of "consisting essentially of' and in a further preferred embodiment the meaning of "consisting of'.

Further preferred embodiments of the invention are subject of the subclaims.

The present invention is further illustrated by way of the following figures.

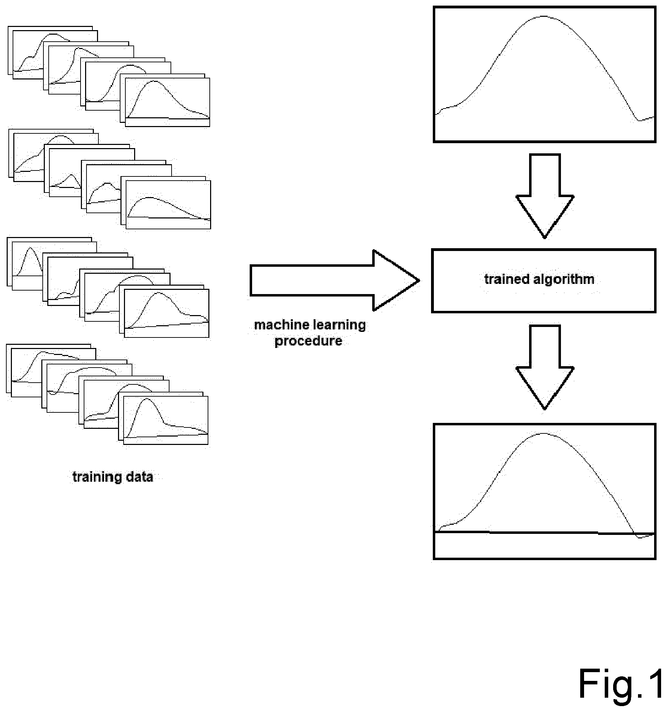

**Fig. 1** shows a flowchart of the individual steps of the method for determining a baseline in a blood glucose curve according to the present invention. Initially, an algorithm is trained to automatically determine baselines in blood glucose curves based on training data comprising blood glucose curves of individuals having an impact factor-accounting baseline. Subsequently, the trained algorithm is applied on a blood glucose curve of an individual to obtain a blood glucose curve having an automatically determined baseline.

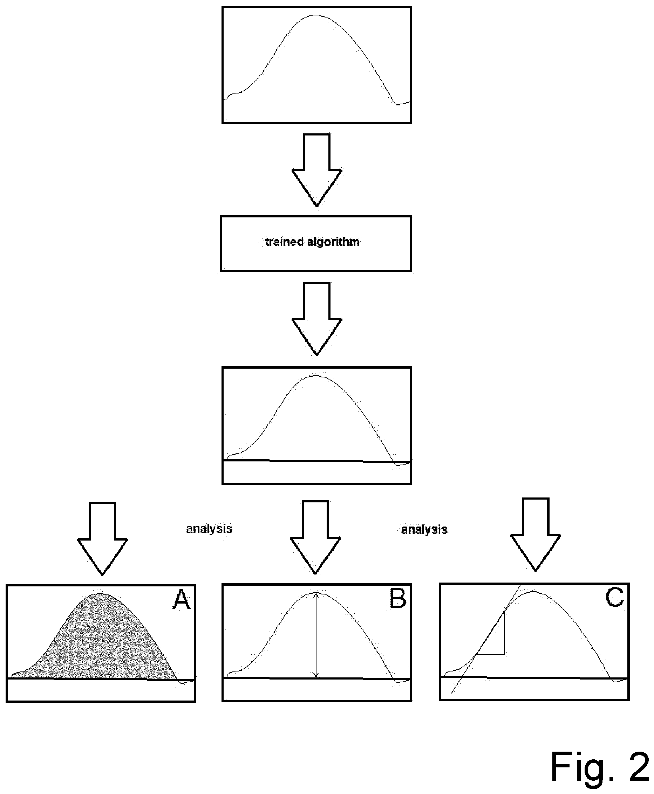

**Fig. 2** shows a flowchart of the individual steps of the method for determining a blood glucose response of an individual to at least one impact factor according to the present invention. In the first step of the method, at least one blood glucose curve of an individual in response to at least one impact factor is provided. Subsequently, the trained algorithm obtained by the method for determining a baseline in a blood glucose curve in response to at least one impact factor according to the present invention is applied on the at least one blood glucose curve of the individual so as to obtain a blood glucose curve having an automatically determined baseline. Finally, the obtained blood glucose curve having an automatically determined baseline is analysed to determine the blood glucose response of the individual to the at least one impact factor, such as by calculating the AUC, in particular the iAUC (A; grey shaded), by determining the maximum increase in the blood glucose level relative to the automatically determined blood glucose baseline (B) or by determining the slope of the blood glucose curve (C).

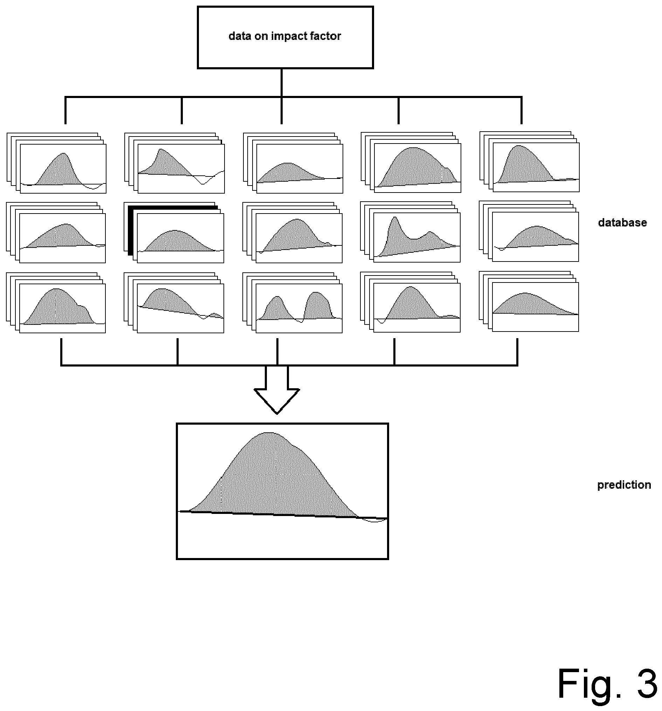

**Fig. 3** shows a flowchart of the individual steps of the method for predicting the blood glucose response of an individual to at least one impact factor according to the present invention. According to the method, data pertaining to at least one impact factor are provided and assigned to at least one blood glucose response in a database comprising blood glucose responses of the individual to different impact factors, preferably comprising blood glucose responses of different individuals to different impact factors, in particular comprising blood glucose responses of different individuals classified into the same nutritype to different impact factors, wherein each of the blood glucose responses in the database has been obtained by the method for determining a blood glucose response of an individual to at least one impact factor according to the present invention and consequently has an automatically determined baseline. Subsequently, based on the assignment to at least one blood glucose response in the database a prediction for a blood glucose response to the at least one impact factor in question is provided. Fig. 3 exemplifies an embodiment of the present invention in which the blood glucose responses in the database have been determined by calculating the AUC, in particular the iAUC (grey shaded). Similarly, other methods for the determination of the blood glucose responses in the database, such as determining the maximum increase in the blood glucose level relative to the automatically determined blood glucose baseline or determining the slope of the blood glucose curve are also within the scope of the present invention.

## Claims

1. A method for determining a baseline in a blood glucose curve, the method comprising:
a) providing training data comprising blood glucose curves of individuals comprising an impact factor-accounting baseline,
b) subjecting the training data provided in step a) to a machine learning procedure to obtain a trained algorithm for automated determination of baselines in blood glucose curves, and
c) determining a baseline in a blood glucose curve by applying the trained algorithm on a blood glucose curve in response.

2. The method of claim 1, wherein the training data provided in step a) contain at least 10, preferably at least 50, preferably at least 100, preferably at least 250, preferably at least 500, preferably at least 750, preferably at least 1000, pairs of blood glucose curves in response to at least one impact factor.

3. The method according to any one of the preceding claims, wherein the machine learning procedure is a supervised machine learning procedure.

4. A method for determining a blood glucose response of an individual to at least one impact factor, the method comprising:
aa) providing at least one blood glucose curve of the individual in response to at least one impact factor,
bb) applying the trained algorithm obtained in step b) of the method according to any one of claims 1 to 3 on the at least one blood glucose curve provided in step aa) to obtain at least one blood glucose curve of the individual having an automatically determined baseline, and
cc) analysing the at least one blood glucose curve obtained in step bb) to determine the blood glucose response of an individual to the at least one impact factor.

5. A method for predicting the nutritype of an individual, the method comprising:
i) providing at least one blood glucose response of an individual to at least one impact factor obtained by the method according to claim 4,
ii) assigning the blood glucose response of the individual to the at least one impact factor to a group of blood glucose responses of different individuals to the at least one impact factor in a database using a nutritype classification model, and
iii) outputting the nutritype of the individual based on the at least one blood glucose response of the individual to the at least one impact factor.

6. A method for predicting the blood glucose response of an individual to at least one impact factor, the method comprising:
x) providing data pertaining to at least one impact factor,
y) assigning the data pertaining to at least one impact factor to at least one blood glucose response in a database comprising blood glucose responses of the individual to different impact factors obtained by the method according to claim 4, and
z) outputting a predicted blood glucose response of the individual to the at least one impact factor.

7. The method according to any one of the preceding claims, wherein the at least one impact factor is selected from food intake, physical activity, mental activity, stress, health condition medication, sleep or a combination thereof.

8. A computer program product, directly loadable into the internal memory of a digital computer, comprising software code portions which, when the program is executed by a computer cause the computer to carry out i) the method according to any one of claims 1 to 3, ii) the method according to claim 4, iii) the method according to claim 5, iv) the method according to claim 6 and/or v) the method according to claim 12.

9. A computer-readable storage medium comprising software code portions, which when executed by a computer cause the computer to carry out i) the method according to any one of claims 1 to 3, ii) the method according to claim 4, iii) the method according to claim 5, iv) the method according to claim 6 and/or v) the method according to claim 12.

10. A device comprising:
- a display unit, displaying a user interface,
- an input unit,
- a memory unit, and
- a processing unit,
wherein the memory unit comprises a computer program product according claim 8.

11. The device according to claim 10, wherein the device is a mobile device, in particular a battery-powered wireless mobile device.

12. A method for determining personalized lifestyle recommendations for an individual, the method comprising the steps:
i) providing a database comprising blood glucose responses of the individual to different impact factors determined by the method according to claim 4,
ii) providing data pertaining to at least one impact factor affecting the individual,
iii) calculating personalized lifestyle recommendations for the individual based on the blood glucose response of the individual to the at least one impact factor affecting the individual in the database provided in step i), and
iv) outputting personalized lifestyle recommendations for the individual.

13. The method according to claim 12, wherein the method is a method for determining the composition of a personalized diet, wherein in step iii) the composition of a personalized diet based on the blood glucose response of the individual to the at least one impact factor affecting the individual in the database provided in step i) is calculated, and wherein in step iv) the composition of a personalized diet is put out.

14. A method for preparing a personalized diet, the method comprising the steps:
xx) determining the composition of a personalized diet according to the method according to claim 13, and
yy) preparing the components of the personalized diet having the composition determined in step xx).

15. A personalized diet obtained by the method of claim 14 for use in the treatment of blood glucose related diseases and/or disorders.

16. The personalized diet for use according to claim 14, wherein the blood glucose related disease and/or disorder is selected from the group consisting of diabetes mellitus type 1 (DMT1), diabetes mellitus type 2 (DMT2), gestational diabetes, hyperglycaemia, metabolic syndrome, cardiovascular diseases, glucose intolerance, polycystic ovary syndrome (PCOS), migraine, non-alcoholic fatty liver disease (NAFLD), cancer, acne, atopic dermatitis, psoriasis, rosacea, atrial fibrillation, dyslipidaemia, HIV, arterial hypertension, pre-diabetes, obesity, brain/cognitive dysfunction, Alzheimer's disease, depression, cartilage damage, Parkinson's disease, rheumatic diseases, chronic inflammation.

17. Use of a personalized diet obtained by the method of claim 14 in induction of pregnancy, regulation of menstrual cycle, weight loss, anti-aging or in treatment and/or prevention of menstrual problems and symptoms of menopause.
